# FASCICULE DE BREVET EUROPEEN

(11) **EP 3 188 680 B1**
(45) Date de publication et mention de la délivrance du brevet: **09.01.2019**
(21) Numéro de dépôt: 15753087.4
(22) Date de dépôt: 01.07.2015
(51) Int. Cl.: A61B 18/14

(54) **DISPOSITIF ELECTRO-CHIRURGICAL POLYVALENT**
MULTIFUNKTIONALE ELEKTROCHIRURGISCHE VORRICHTUNG
MULTIFUNCTIONAL ELECTROSURGICAL DEVICE

(30) Priorité: 01.09.2014 FR 1458158
(43) Date de publication de la demande: 12.07.2017
(73) Titulaire: Sarrazin, Franck, 49320 Charce Saint Ellier (FR); Rousseau, Nicolas, 33410 Omet (FR)
(72) Inventeur: ROUSSEAU, Nicolas, 33410 Omet (FR)
(74) Mandataire: IP Trust
(86) Numéro de dépôt international: PCT/FR2015/051812
(87) Numéro de publication internationale: WO 2015/193627

(56) Documents cités:
- WO-A1-02/03874
- DE-A1-102004 031 141
- US-A1- 2004 049 185
- US-A1- 2007 179 499
- US-A1- 2014 148 801

## Description

La présente invention se rapporte à un dispositif électro-chirurgical polyvalent.

### ARRIERE PLAN TECHNOLOGIQUE DE L'INVENTION

On connaît de l'état de la technique, des dispositifs électro-chirurgicaux permettant de réaliser des opérations d'incision ou de cautérisation des tissus d'un patient.

Il s'agit généralement de dispositifs guidés à la main par un chirurgien permettant d'appliquer aux tissus une énergie haute fréquence, issue d'un générateur, par l'intermédiaire d'une première électrode. Le générateur permet de choisir le niveau d'énergie et la forme d'onde adaptés à l'intervention. En configuration mono polaire, une électrode de retour est positionnée sur le patient. En configuration bipolaire, le dispositif est muni d'une seconde électrode pour permettre de faire circuler l'énergie dans les tissus, entre les deux électrodes.

Les opérations d'incision et/ou de cautérisation sont produites par la chaleur susceptible de se développer au niveau des tissus où se concentre et/ou circule l'énergie.

Suivant la densité d'énergie délivrée par la première électrode un effet d'incision ou de cautérisation est obtenu. D'une manière générale, l'incision est réalisée lorsque la densité d'énergie est suffisante pour détruire les cellules formant les tissus. Le niveau d'énergie et la forme d'onde fournis par le générateur sont ajustables par le chirurgien à l'aide d'interrupteurs de commande disposés, par exemple, sur le dispositif ou au sol ou en intervenant directement sur le générateur.

Des dispositifs connus de l'état de la technique permettent d'opérer dans un premier mode dit «de pression ». Dans ce mode, le dispositif électro-chirurgical est appliqué en contact guidé sur le tissu, c'est-à-dire en maintenant le contact entre l'électrode, ou les électrodes, et les tissus au cours du déplacement guidé du dispositif par le chirurgien. Le document US5833689 présente un tel dispositif permettant, selon le niveau d'énergie et/ou la forme d'onde délivré par le générateur, d'inciser ou de cautériser les tissus traités.

D'autres dispositifs connus de l'état de la technique permettent d'opérer dans un second mode dit «de préhension ». Dans ce mode, les tissus sont saisis entre les deux électrodes du dispositif qui se présente alors sous la forme d'une pince ou de ciseaux. Les documents US20140148801 et US2004049185 présentent un tel dispositif qui permet, selon le niveau d'énergie et/ou la forme d'onde délivrés par le générateur, d'inciser ou de cautériser les tissus saisis.

Au cours d'une opération, le chirurgien dispose généralement d'une multiplicité de ces dispositifs électro-chirurgicaux permettant, selon le besoin, d'opérer dans un mode de pression ou dans un mode de préhension. Les changements répétés de dispositifs électro-chirurgicaux pour passer d'un mode à l'autre, et l'ajustement répété de l'énergie délivrée par le générateur pour commuter le dispositif entre incision et cautérisation constituent des contraintes importantes pour le chirurgien. Ces contraintes imposent en effet d'interrompre régulièrement l'opération, ce qui est une source de risques et d'inefficacité pour son bon déroulement, et mobilisent de plus des ressources matérielles et humaines coûteuses. Elles conduisent par ailleurs à introduire et extraire de manière répétée le dispositif électro-chirurgical adapté pour accéder aux tissus et les traiter, ce qui peut former une source de difficultés ou de risques accrus.

### OBJET DE L'INVENTION

Un but de l'invention est de proposer un dispositif électro-chirurgical polyvalent permettant de traiter des tissus dans plusieurs modes de fonctionnement différents, et notamment en pression et en préhension.

Un autre but de l'invention est de proposer un dispositif électro-chirurgical polyvalent pouvant traiter des tissus en incision et en cautérisation sans nécessairement ajuster le niveau d'énergie et/ou la forme d'onde délivrés par le générateur pendant l'intervention.

### BREVE DESCRIPTION DE L'INVENTION

En vue de la réalisation de l'un au moins de ces buts, l'objet de l'invention propose un dispositif électro-chirurgical polyvalent pour le traitement de tissus biologiques selon la revendication indépendante 1.

Le dispositif selon l'invention est remarquable en ce que les électrodes sont configurées pour permettre la formation du premier contact électrique sur la surface externe de la première électrode et la formation du second contact électrique sur la partie en saillie de la seconde électrode.

Ainsi, il est possible de traiter les tissus en préhension en les saisissant entre chacune des électrodes des membres articulés.

De plus, la forme concave de la première électrode et la position en saillie de la seconde électrode vis-à-vis de la première électrode lorsque les deux membres sont rapprochés l'un de l'autre, permet d'établir et de maintenir le contact électrique entre la surface externe de la première électrode et les tissus et entre la partie en saillie de la seconde électrode et les tissus au cours du déplacement guidé du dispositif et de traiter ainsi les tissus en pression.

Selon d'autres caractéristiques avantageuses et non limitatives de l'invention, prises seules ou en combinaison :
- la seconde électrode n'est pas entièrement logée, sur sa hauteur, à l'intérieur de la concavité de la première électrode lorsque les deux membres sont rapprochés l'un de l'autre, de sorte que la seconde électrode dépasse en hauteur de cette concavité.
- les axes longitudinaux des électrodes ne sont pas parallèle l'un à l'autre, lorsque les deux membres sont rapprochés l'un de l'autre.
- la seconde électrode présente une extrémité en forme de lame ou en forme d'aiguille.
- la seconde électrode présente une extrémité en forme de lame et est orientée de sorte que sa tranche se loge dans la première électrode présentant une section transversale concave, lorsque les deux membres sont rapprochés l'un de l'autre.
- le second membre est pourvu de moyens permettant de déplacer en rotation la seconde électrode autour d'un axe sensiblement défini par l'axe longitudinal du second membre.
- les moyens permettant de déplacer en rotation la seconde électrode comprennent une rosace.
- le dispositif est muni de moyens de guidage du premier et du second membres (2, 3).
- les électrodes sont assemblées à des éléments de saisie de manière interchangeable.

### BREVE DESCRIPTION DES DESSINS

L'invention sera mieux comprise à la lumière de la description qui suit des modes de réalisation particuliers et non limitatifs, en référence aux figures ci-jointes parmi lesquelles :
- la figure 1 représente une vue d'ensemble d'un mode de réalisation du dispositif électro-chirurgical polyvalent selon l'invention;
- la figure 2 représente une vue rapprochée des électrodes du dispositif électro-chirurgical polyvalent dans un mode de réalisation préféré ;
- la figure 3 représente la disposition du dispositif de l'invention pour un traitement d'incision en pression;
- la figure 4 représente une disposition alternative du dispositif de l'invention pour un traitement d'incision en pression ;
- la figure 5 représente la disposition du dispositif de l'invention pour un traitement d'incision en préhension ;
- la figure 6 représente la disposition du dispositif de l'invention pour un traitement de cautérisation en pression ;
- la figure 7 représente la disposition du dispositif de l'invention pour un traitement de cautérisation en préhension.
- la figure 8 représente une configuration particulièrement avantageuse du dispositif électro-chirurgical conforme à l'invention.

### DESCRIPTION DETAILLEE DE L'INVENTION

Le terme « incision » utilisé dans la description détaillée qui suit désigne la réalisation d'une coupure ou d'une entaille de toute dimension et profondeur dans des tissus biologiques. Il peut s'agir d'une coupure ou d'une entaille réalisée au cours d'une opération de dissection. L'incision peut comprendre la coagulation des tissus incisés.

Le terme « cautérisation» quant à lui désigne dans cette description les effets de tout traitement de surface des tissus biologiques, obtenus par application de chaleur. Cette application peut conduire à des phénomènes de coagulation, de dessiccation, d'hémostasie ou à tout autre phénomène conduisant à l'obturation de vaisseaux sanguins.

Par « traitement », on désigne indifféremment une incision ; une cautérisation ou tout autre effet que le dispositif selon l'invention pourrait provoquer sur les tissus sur lesquels il est appliqué.

Finalement, le terme « tissus » désigne tout ensemble de cellules biologiques ; formant tout ou partie d'un organe ou d'un corps.

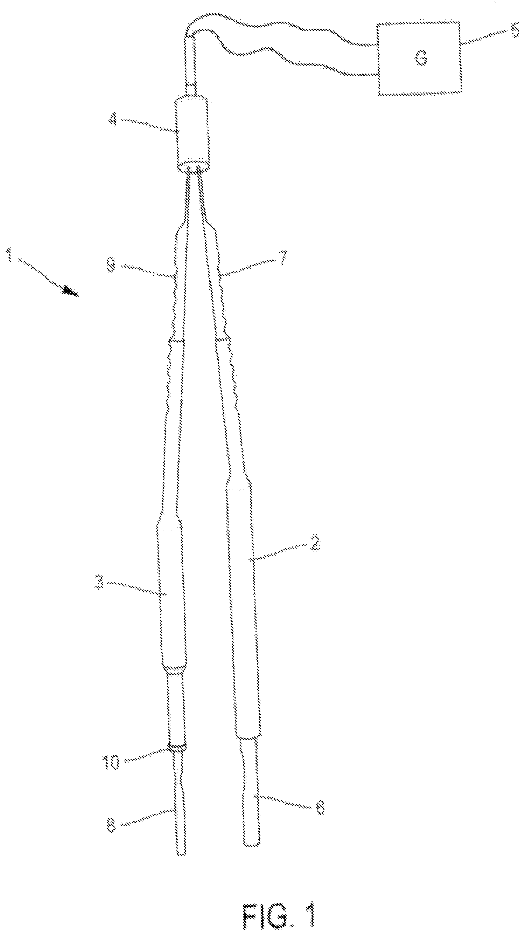

La figure 1 représente une vue d'ensemble d'un mode de réalisation du dispositif électro-chirurgicale polyvalent 1 selon l'invention. Il comprend un premier membre 2 et un second membre 3 reliés l'un à l'autre par un pivot d'articulation 4 permettant d'approcher et d'éloigner les membres 2, 3 l'un de l'autre.

Dans la configuration préférée du dispositif électro-chirurgical polyvalent 1 représenté sur la figure 1, le pivot d'articulation 4 est disposé à l'extrémité arrière de chaque membre 2, 3 pour former une pince. D'autres configurations sont possibles, et notamment le pivot d'articulation 4 peut être placé dans la partie centrale des membres 2, 3 pour former des ciseaux.

Les membres 2, 3 sont maintenus éloignés l'un de l'autre lorsqu'aucune pression n'est exercée sur ceux-ci, à l'aide, par exemple, de moyens de retenue incorporés dans le pivot d'articulation 4.

Le dispositif électro chirurgical polyvalent 1 est électriquement relié à un générateur 5, connu en soi, et apte à générer les signaux hautes fréquences conduisant à la réalisation des traitements, tels qu'une incision et/ou une cautérisation. Il peut s'agir d'un générateur de courant.

Le premier membre 2 du dispositif électro-chirurgical polyvalent 1 est constitué en son extrémité avant d'une première électrode 6 assemblée à un élément de saisie 7. Cette première électrode 6 est en liaison électrique avec le générateur 5 par l'intermédiaire d'un câble courant par exemple le long de l'élément de saisie 7 ou incorporé à celui-ci.

Similairement, le second membre 3 est constitué en son extrémité avant d'une seconde électrode 8 assemblée à un élément de saisie 9. Cette seconde électrode 8 est en liaison électrique avec le générateur 5 par des moyens similaires à ceux décrits en relation avec le premier membre 2.

L'électrode 6 et l'élément de saisie 7, et respectivement l'électrode 8 et l'élément de saisie 9, peuvent être solidaires l'un de l'autre ou assemblés de manière permanente. Dans ce cas, le dispositif électro chirurgical polyvalent 1 est à usage unique. L'électrode 6, 8 et l'élément de saisie correspondant 7,9 peuvent alors être formés d'un unique élément conducteur, par exemple métallique, permettant d'assurer la liaison électrique entre l'électrode 6,8 et le générateur 5. La partie de l'élément métallique correspondant à l'élément de saisie 7, 9 est munie d'une gaine isolante électriquement, afin de permettre la manipulation du dispositif 1 lors de son fonctionnement.

Avantageusement, les électrodes 6, 8 sont assemblées de manière interchangeable aux éléments de saisie 7, 9 ; par exemple par l'intermédiaire de logements formés dans les éléments de saisie 7, 9 dans lesquels les extrémités arrières des électrodes 6, 8 peuvent être insérées, et établir la connexion électrique avec le générateur 5. Dans le cas d'électrodes interchangeables, seules ces électrodes doivent être remplacées régulièrement, ce qui constitue un avantage économique notable. Dans certains cas, il peut être avantageux de stériliser les électrodes 6,8 entre chaque intervention afin de permettre leur réutilisation.

Les électrodes 6, 8 sont constituées, ou comprennent, un matériau électriquement conducteur. Il peut s'agir par exemple d'acier inoxydable. L'acier inoxydable peut par endroit être poli, afin d'éviter l'accroche des tissus sur les électrodes 6, 8 pendant le traitement.

Les éléments de saisie 7, 9 sont quant à eux constitués de matériaux isolants, comme par exemple du polyétheréthercétone (également dénommé PEEK), permettant leur manipulation sans risque par le chirurgien au cours d'une intervention.

Comme indiqué précédemment, les éléments de saisie 7, 9 peuvent être munis de conduits pour loger les câbles assurant la connexion entre chacune des électrodes 6, 8, et le générateur 5.

Alternativement, les éléments de saisie 7, 9 peuvent être formé d'un coeur électriquement conducteur, par exemple un coeur métallique, muni d'une gaine isolante électriquement.

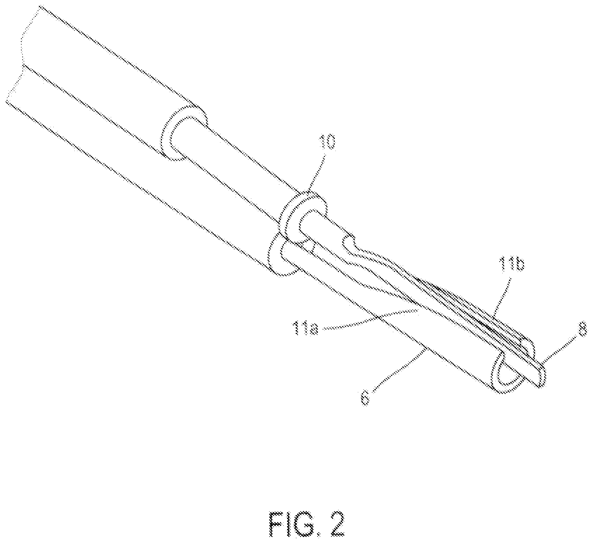

Selon l'invention, et comme particulièrement notable sur les figures 2, 3, 4 et 6 :
- la première électrode 6, associée au premier membre 2, présente une section transversale concave apte à loger en partie la seconde électrode 8 lorsque les deux membres 2, 3 sont rapprochés l'un de l'autre par rotation autour du pivot d'articulation 4 ; La première électrode 6 définit donc un logement concave, présentant une surface interne. La seconde électrode présente des flancs extérieurs 11a, 11b définissant une surface externe, complémentaire à la surface interne.
- la seconde électrode 8 est en saillie de la première électrode 6 lorsque les deux membres 2,3 sont rapprochés l'un de l'autre, afin de permettre le contact de chaque électrode avec les tissus.

Les électrodes sont configurées pour permettre la formation d'un premier contact électrique sur la surface externe de la première électrode 6 et la formation d'un second contact électrique sur la partie en saillie de la seconde électrode 8.

Cette disposition est facilitée en configurant le dispositif 1 pour que les axes longitudinaux des électrodes 6, 8 ne soient pas parallèle l'un à l'autre dans une configuration de travail, lorsque les deux membres 2,3 sont rapprochés l'un de l'autre. Par exemple, et comme cela est apparent sur la figure 1, le dispositif 1 peut être configuré pour que les deux électrodes 6, 8 présentent des axes longitudinaux parallèles l'un à l'autre en position de repos, c'est à dire lorsqu'aucune pression n'est appliqué à l'un des membres 2, 3. Ceci peut être obtenu en donnant une forme courbe aux éléments de saisie 7, 9 selon leur axes longitudinaux, et selon une direction de courbure opposée l'une à l'autre.

Ainsi, dans une première configuration, l'extrémité de la seconde électrode 8 dépasse en longueur l'extrémité de la première électrode et ainsi former la partie en saillie. Le dépassement en longueur peut être compris entre 0,01 à 20mm.

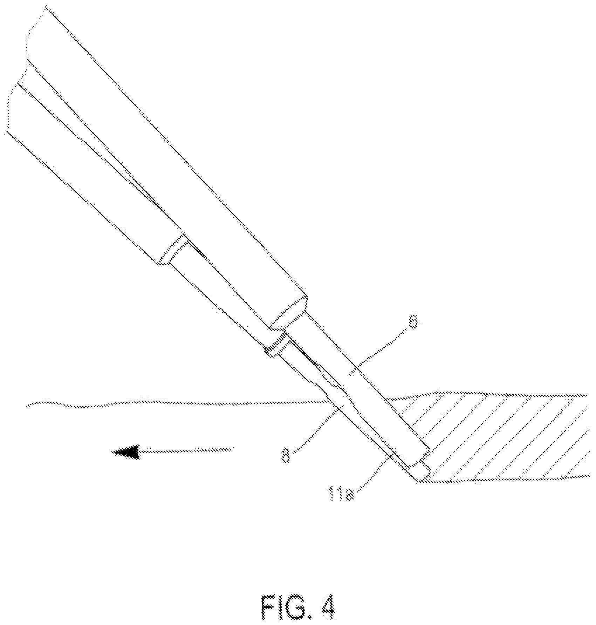

Dans une seconde configuration, alternative ou complémentaire à la première configuration, la seconde électrode 8 n'est pas entièrement logée dans la concavité de la première électrode 6, et elle dépasse en hauteur les flancs 11a, 11b de la concavité de la première électrode 6, pour former la partie en saillie. Cette configuration permet donc également de mettre la seconde électrode 8 en saillie de la première électrode 6 lorsque les deux membres 2,3 sont rapprochés l'un de l'autre, afin de permettre le contact de chaque électrode avec les tissus. Cette disposition est particulièrement visible sur la figure 4, et permet une mise en contact linéaire de l'électrode 8 avec les tissus, comme cela sera exposé par la suite. Le dépassement en hauteur peut être compris entre 0,01 à 20mm.

La forme concave de la première électrode 6 présente l'avantage de permettre le rapprochement à très courte distance des électrodes 6, 8 et de faciliter de la sorte la circulation de l'énergie dans les tissus. De plus, les flancs 11a, 11b de la première électrode 6 forment des surfaces de contacts électriques étendues avec les tissus qui peuvent être mises à profit lors de l'utilisation du dispositif, ainsi que cela sera détaillé par la suite. Et en conséquence, la surface du premier contact formé entre la première électrode 6 et les tissus peut être plus étendue que la seconde surface de contact formée entre la seconde électrode 8 et les tissus. Dans ce cas, la densité d'énergie est plus intense au niveau de la seconde électrode 8, ce qui la rend active au cours du traitement, qu'au niveau de la première électrode 6, ce qui la rend passive au cours du traitement.

Dans une variante, l'extrémité de la première électrode peut être biseauté, et la section en biseau former et/ou participer également à la surface du premier contact avec les tissus.

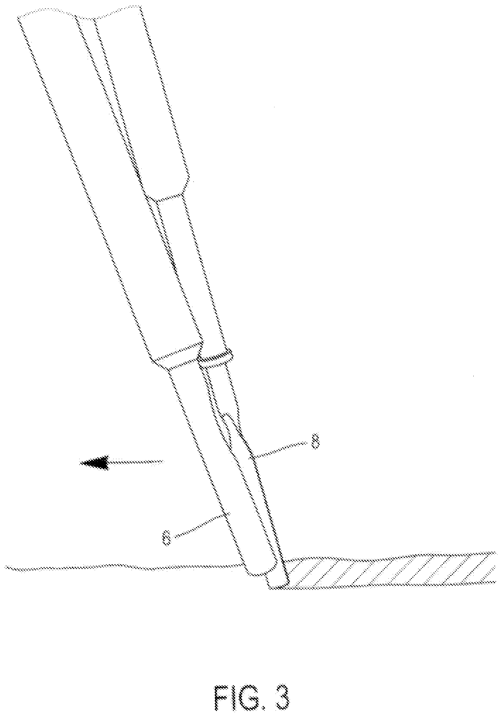

Ainsi, le dispositif électro-chirurgical polyvalent 1 peut être employé dans un premier mode de pression. Ce premier mode est obtenu en exerçant une pression sur les éléments de saisie 7,9 de manière à rapprocher les membres 2, 3, par déplacement en rotation autour du pivot d'articulation 4. Comme cela est visible sur les figures 3, 4 et 6, la disposition en saillie de la seconde électrode 8 vis-à-vis de la première électrode 6 permet de former les contacts électriques respectifs avec les tissus en ajustant l'angle de travail du dispositif 1. Dans ce mode d'utilisation, le premier contact électrique est formé sur la surface externe de la première électrode 6 et la formation du second contact électrique sur la partie en saillie de la seconde électrode 8.

L'angle de travail du dispositif correspond à l'angle existant entre son axe longitudinal et le plan de travail formé par le plan tangent aux tissus traités. Suivant la pression exercée par le chirurgien sur les membres de saisie 7,9, il est possible d'ajuster le rapprochement de la seconde électrode 8 vis-à-vis de la première électrode 6 ; et en conséquence d'ajuster l'angle de travail du dispositif 1. Le chirurgien peut de la sorte choisir l'angle de travail le plus propice au traitement à engager.

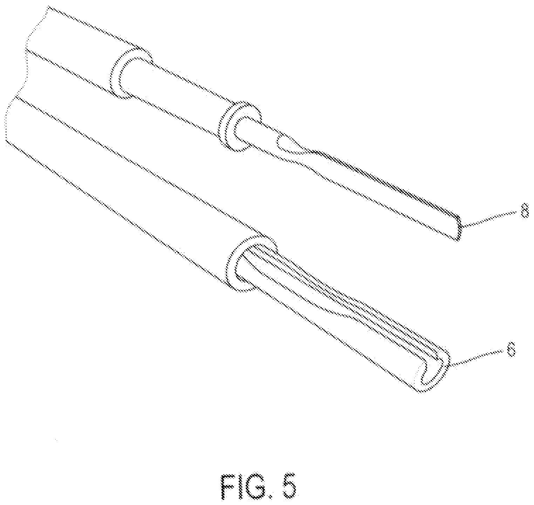

Le dispositif électro-chirurgical polyvalent 1 selon l'invention peut-être également employé dans un deuxième mode de préhension, représenté sur la figure 5, le pivot d'articulation 4 permettant d'utiliser les membres 2,3 pour pincer les tissus entre les électrodes 6, 8, et de former les contacts électriques entre ces électrodes 6, 8, et les tissus en vue de les traiter.

Il est ainsi possible de disposer d'un unique dispositif pour traiter en pression et en préhension les tissus biologiques au cours d'une intervention chirurgicale.

Dans une configuration particulière, la section transversale concave de la première électrode 6 est une portion de cercle. Il est ainsi possible de former des surfaces de contact importantes sur les flancs 11a, 11b de cette électrode pour un encombrement minimum de l'électrode 6.

La seconde électrode 8 peut avoir une forme quelconque, par exemple une forme d'aiguille, mais dans le mode de mise en oeuvre préféré du dispositif 1 de l'invention, la seconde électrode 8 du second membre 3 présente une extrémité en forme de lame. Par « forme de lame », on signifie en forme de bande, plat et mince.

Ainsi, suivant l'orientation du dispositif 1 et/ou de la seconde électrode 8, il est possible de former le second contact électrique entre la seconde électrode 8 et les tissus soit sur le plat, soit sur la tranche de la seconde électrode 8 en forme de lame. On peut varier ainsi l'étendue de la surface de contact, et en conséquence la densité d'énergie délivrée et/ou circulant au niveau du second contact électrique et dans son voisinage, sans nécessiter d'ajustement du niveau d'énergie et la forme d'onde délivrés par le générateur 5. Comme cela sera détaillé par la suite, le dispositif 1 peut traiter les tissus en incision et en cautérisation.

Il n'est pas nécessaire que la seconde électrode 8 présente un bord tranchant. En effet, l'incision des tissus est obtenue par l'échauffement produit par la circulation d'énergie avec une densité suffisante dans ceux-ci, et non par effet de cisaillement.

Lorsque les deux membres 2, 3 sont rapprochés l'un de l'autre par rotation autour du pivot d'articulation 4, il est préférable que les deux électrodes 6, 8 n'entrent pas en contact électrique direct. Cette absence de contact électrique direct peut être obtenue en évitant de trop rapprocher les deux membres l'un de l'autre lors de l'utilisation du dispositif. En mode de préhension, l'absence de contact électrique direct est obtenue par la présence des tissus entre les électrodes 6, 8. Il peut être également obtenu en ajustant les dimensions et les positions de l'une et/ou de l'autre des électrodes 6, 8, de sorte que lorsque les deux membres 7, 9 viennent en butée, les deux électrodes 6, 8 ne se contactent pas. A cet effet, l'un des membres 2,3 peut présenter un dispositif de butée, comme par exemple une collerette 10 à l'extrémité de l'un des éléments de saisie 7,9.

Alternativement ou en complément de ces différents moyens, la surface de la première électrode 6, et notamment la surface interne de la concavité de logement, susceptible d'entrer en contact avec la seconde électrode 8 lorsque les deux membres 2, 3 sont rapprochés l'un de l'autre, peut être munie d'un revêtement électriquement isolant.

Ainsi, et à titre d'exemple, la première électrode 6 peut être formée en acier inoxydable, la surface intérieure de la concavité de cette électrode 6 étant munie d'un revêtement isolant électriquement.

Le dispositif peut également être muni de moyens de guidage du premier et du second membre lors de leur rapprochement. Ces moyens permettent notamment de positionner avec précision une partie au moins de la seconde électrode 8 dans la concavité de la première électrode 6. Il peut s'agir par exemple d'un ergot positionné sur l'un des membres 2, 3 coulissant dans une ouverture formée en vis-à-vis de l'ergot dans l'autre membre, lorsque l'on rapproche les deux membres 2, 3 l'un de l'autre.

De manière avantageuse, le second membre 3 dispose de moyens permettant de déplacer en rotation la seconde électrode 8 autour d'un axe sensiblement défini par l'axe longitudinal du second membre 3. Ces moyens peuvent être mis en oeuvre par le chirurgien de sorte qu'il puisse passer d'une configuration où le plat de la seconde électrode 8 est orienté pour électriquement contacter les tissus, à une configuration où la tranche de la seconde électrode 8 est orientée pour électriquement contacter les tissus. Il peut s'agir d'un ergot formé sur la seconde électrode 8, permettant de déplacer en rotation, et préférentiellement de 90°, la seconde électrode 8 dans le logement de l'élément de saisie 9 dans laquelle elle est insérée.

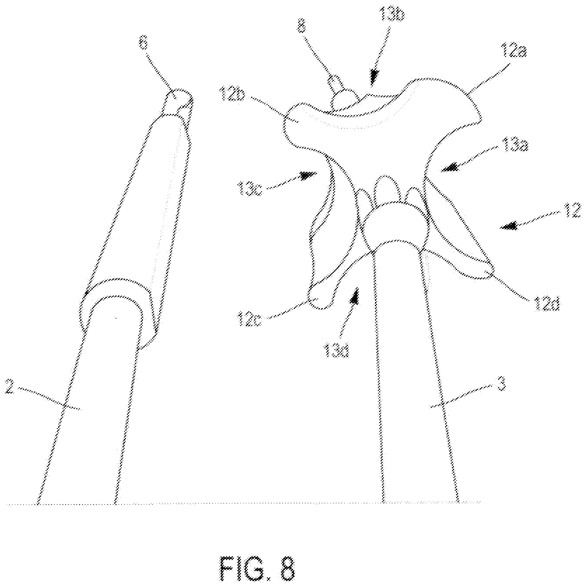

La figure 8 représente une configuration particulièrement avantageuse du dispositif électro-chirurgical 1. Dans cette configuration, le second membre 3 dispose de moyens permettant de déplacer en rotation la seconde électrode 8 sous la forme d'un élément en rosace 12, présentant 4 ailes 12a, 12b, 12c, 12d et définissant 4 encoches 13a, 13b, 13c, 13d permettant de recevoir le premier membre 2 lorsque les deux membres 2, 3 sont rapprochés l'un de l'autre. Les ailes et les encoches contiguës sont séparées l'une de l'autre d'une ouverture angulaire de 90°. L'élément en rosace est libre de se mouvoir en rotation autour d'un axe sensiblement défini par l'axe longitudinal du second membre 3, et entraine en rotation la seconde électrode 8. Ce mouvement de rotation est aisément réalisé par le chirurgien en agissant sur une des ailes 12a, 12b, 12c, 12d lorsque les deux membres 2, 3 ne sont pas rapproché l'un de l'autre, au cours de son intervention, pour placer la seconde électrode selon une orientation désirée. La forme évasée des encoches 13a, 13b, 13c, 13d constitue les moyens de guidage du premier et du second membre 3 lors du rapprochement des deux membres 2, 3 du dispositif 1. Le fond de chaque encoche encoches 13a, 13b, 13c, 13d constitue également une buté, pour le second membre 2 lorsque les deux membres 2, 3 sont rapprochés l'un de l'autre. La position du fond dans chaque encoche, et donc la profondeur de chaque encoche peut être choisie pour positionner la seconde électrode 8 à une distance choisie de la première électrode 6 lorsque les deux membres 2, 3 sont rapprochés l'un de l'autre et placés en buté. Cette distance peut être différente suivant l'orientation choisie de la seconde électrode 8.

Le dispositif électro chirurgical polyvalent 1 peut être muni d'un ou plusieurs interrupteurs de commande, par exemple disposés sur l'un de ses membres 2, 3, pour connecter les électrodes 6, 8 au générateur 5, et mettre en fonctionnement le dispositif 1. Alternativement, le ou les interrupteurs de commande peuvent être prévus sur le générateur 5 ou sur la connexion électrique entre le générateur 5 et le dispositif 1. Il peut s'agir également, d'un ou plusieurs interrupteurs de commande au sol que le chirurgien peut commuter du pied au cours de l'intervention. Outre la mise en fonctionnement du dispositif 1, les interrupteurs peuvent également permettre de commander le niveau d'énergie ou la forme d'onde délivrée par le générateur 5.

Mais l'invention ne nécessite pas l'existence de plusieurs interrupteurs de commande car le chirurgien peut aisément choisir la densité d'énergie appliquée aux tissus, en modifiant l'étendue de la surface de contact d'une ou des électrodes 6, 8 avec les tissus comme cela sera détaillé en relation avec la description des figures 3 à 7.

Une méthode de traitement de tissus biologiques est également divulguée, et notamment une incision et/ou une cautérisation, comprenant les étapes suivantes :
- déplacer l'un vers l'autre une première électrode 6 présentant une section transversale concave d'un premier membre 2 et une seconde électrode 8 d'un second membre 3 du dispositif électro-chirurgical polyvalent 1 de manière à loger en partie la seconde électrode 8 dans la concavité de la première électrode 6 et mettre en saillie la seconde électrode 8 de la première électrode 6 ;
- former un premier contact entre les tissus et une surface externe de la première électrode 6 du premier membre 2;
- former un second contact électrique entre les tissus et la seconde électrode 8 du second membre 3 ;
- faire circuler une énergie dans les tissus entre le premier contact électrique et le second contact électrique pour réaliser le traitement.

Les trois premières étapes de formation des contacts électriques et de déplacement des électrodes 6,8 peuvent être réalisées dans un ordre quelconque voire même simultanément.

L'étape de déplacement l'un vers l'autre des électrodes 6,8 permet de placer en saillie la seconde électrode 8 vis-à-vis de la première électrode 6, en particulier lors d'une incision;

Le déplacement de la première électrode 6 et de la seconde électrode 8 l'un vers l'autre est obtenu par rotation autour du pivot d'articulation 4 du premier membre 2 et du second membre 3.

Préférentiellement, le second contact électrique est formé entre les tissus et la partie en saillie de la seconde électrode 8.

Le premier mode de mise en oeuvre de la méthode de traitement concerne une méthode d'incision par pression. Dans ce premier mode, le premier contact électrique est réalisé sur la surface externe de la première électrode 6. Le second contact électrique est réalisé sur la tranche de la seconde électrode 8, en forme de lame, en son extrémité en saillie. Cette configuration du dispositif 1 est représentée sur la figure 3. Sur cette figure, le dispositif 1 est déplacé dans le sens de la flèche, et les tissus qui ont été parcourus par le dispositif 1 et incisés sont représentés sous une forme hachurée.

Si cela est nécessaire, ce premier mode de mise en oeuvre peut nécessiter une étape préalable d'orientation de la seconde électrode 8 par rotation de cette électrode autour d'un axe sensiblement longitudinal au second membre 3, afin d'assurer que le second contact électrique s'effectue bien sur la tranche de la seconde électrode 8.

Le second contact électrique s'effectue dans ce mode de mise en oeuvre sur une surface ponctuelle, réduite avec les tissus. En conséquence, l'énergie déposée au niveau du second contact électrique et/ou circulant dans les tissus entre le premier contact électrique et le second contact électrique présente une densité importante, conduisant à l'incision des tissus. A ce titre, ce premier mode de mis en oeuvre n'est pas limité à une seconde électrode 8 présentant une extrémité en forme de lame, et toute électrode 8 présentant une extrémité assurant un contact électrique ponctuel ou quasi ponctuel convient. Il peut s'agir par exemple d'une électrode 8 présentant une extrémité en forme d'aiguille.

Dans ce mode de mise en oeuvre, la méthode peut également comprendre une étape supplémentaire de déplacement du premier et du second contact électrique sur les tissus pendant l'opération afin de former l'incision suivant une longueur et un parcours choisi. Ce déplacement est obtenu en déplaçant le dispositif électro-chirurgical polyvalent 1 en contact guidé sur les tissus, selon par exemple, la direction indiquée par la flèche de la figure 3.

Une alternative à ce premier mode de réalisation est présentée sur la figure 4, dans lequel le dispositif est sensiblement retourné de 180 degrés par rapport au mode de réalisation précédent. Dans cette alternative, le premier contact électrique est réalisé sur la première électrode 6, au niveau des flancs 11a, 11b de la concavité (notamment visibles sur la figure 2). Le second contact est réalisé sur la tranche en saillie de la seconde électrode 8. La surface de contact, linéaire, entre la seconde électrode 8 et les tissus s'étend sur une portion de la tranche de l'électrode 8, ce qui permet d'inciser les tissus sur une plus grande longueur sans nécessairement déplacer le dispositif 1. Cette alternative permet également de former des incisions profondes, notamment lorsque le niveau d'énergie délivré par le générateur est augmenté. Sur la figure 4, le dispositif 1 est déplacé dans le sens de la flèche, et les tissus qui ont été parcourus par le dispositif 1 et incisés sont représentés sous une forme hachurée.

Cette alternative n'est pas limitée à une seconde électrode 8 présentant une extrémité en forme de lame. La seconde électrode 8 peut notamment présenter une extrémité en forme d'aiguille.

Un deuxième mode de mise en oeuvre de la méthode de traitement concerne une méthode d'incision par préhension. Dans ce deuxième mode, les tissus à traiter sont préalablement saisis entre la première électrode 6 et la seconde électrode 8 à l'aide des membres 7, 9, lors de l'étape de déplacement l'un vers l'autre de ces membres.

Comme dans le premier mode de réalisation, et si cela est nécessaire, une étape préalable d'orientation de la seconde électrode 8 peut être prévue.

Ainsi que représenté sur la figure 5, le premier contact est réalisable entre les tissus et la première électrode 6, notamment sur la surface externe et le second contact électrique est réalisable entre les tissus et la tranche de la seconde électrode 8.

Similairement au mode de réalisation précédent, le deuxième contact s'effectue sur une surface réduite avec les tissus au niveau de la seconde électrode, ponctuelle ou linéaire, engendrant une densité d'énergie dans les tissus suffisante pour son incision. Ce mode de réalisation est également compatible avec une seconde électrode 8 présentant une extrémité en forme d'aiguille.

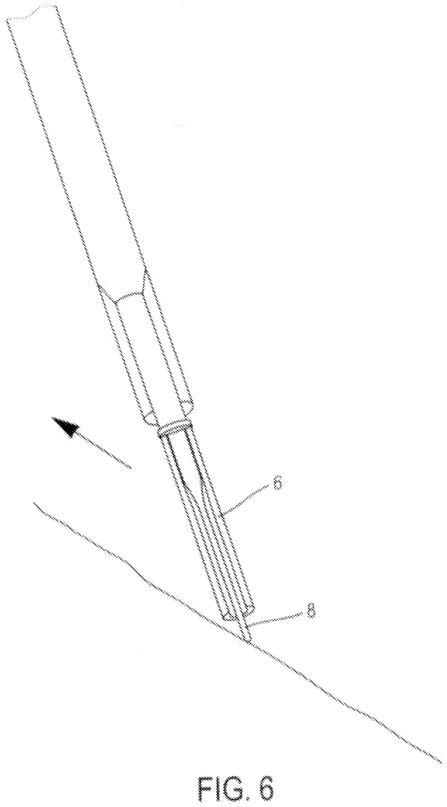

Un troisième mode de mise en oeuvre de la méthode de traitement concerne une méthode de cautérisation en pression. Cette méthode se différencie du premier mode de mise en oeuvre de la méthode de traitement en ce que le second contact électrique entre les tissus et la seconde électrode 8 s'effectue sur le plat de la seconde électrode 8. A cette fin, le dispositif 1 est tourné de 90° par rapport au premier mode de mie en oeuvre. Le premier contact électrique est réalisé entre les tissus et un flanc 11a, 11b de la première électrode 6. Cette disposition est représentée sur la figure 6. Sur cette figure, le dispositif 1 est déplacé dans le sens de la flèche.

Si cela est nécessaire, ce troisième mode de mise en oeuvre peut nécessiter une étape préalable d'orientation de la seconde électrode 8 afin d'assurer que le second contact électrique s'effectue bien sur son côté plat.

Le second contact électrique s'effectue avec les tissus sur une surface importante, sur la partie en saillie de la seconde électrode 8. En conséquence, l'énergie déposée au niveau de la seconde électrode 8 et/ou circulant entre le premier contact électrique et le second contact électrique présente une densité faible, insuffisante pour provoquer une incision, et conduisant à un traitement par cautérisation.

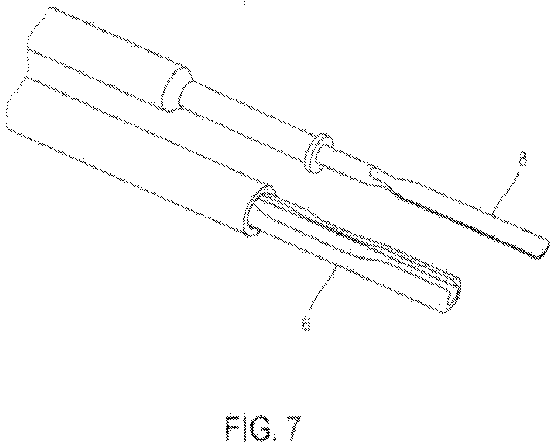

Un quatrième mode de mise en oeuvre de la méthode de traitement concerne une méthode de cautérisation en préhension. Cette méthode se différencie du deuxième mode de mise en oeuvre de la méthode de traitement en ce que le second contact électrique entre les tissus et la seconde électrode 8 s'effectue sur le plat de l'électrode 8. Cette disposition est représentée sur la figure 7. Si cela est nécessaire, ce quatrième mode de mise en oeuvre peut nécessiter une étape préalable d'orientation de la seconde électrode afin d'assurer que le second contact électrique s'effectue bien sur le plat de la seconde électrode 8.

Similairement au troisième mode de mise en oeuvre, le second contact électrique s'effectue avec les tissus sur une surface importante. En conséquence, l'énergie déposée au niveau de la seconde électrode 8 et/ou circulant entre le premier contact électrique et le second contact électrique présente une densité faible, insuffisante pour provoquer une incision, et conduisant à un traitement par cautérisation.

Le chirurgien peut alterner au cours d'une même intervention le déploiement des modes de mise en oeuvre décrit ci-dessus, de manière ininterrompue et avec un unique dispositif. Il est aussi capable de procéder à des interventions d'incision et de cautérisation en passant d'un mode de mise en oeuvre à l'autre, sans nécessiter pendant l'intervention d'ajuster le niveau d'énergie et/ou la forme d'onde délivrés par le générateur 5.

Selon un autre aspect, il est donc divulgué une méthode de traitement de tissus biologiques comprenant les étapes suivantes :
- réaliser un premier traitement des tissus à l'aide du dispositif électro-chirurgical polyvalent 1 relié à un générateur 5;
- modifier en rotation autour de son axe longitudinal l'orientation du dispositif 1 ou d'une partie de ce dispositif 1 ;
- et réaliser un second traitement à l'aide du dispositif 1.

Le premier et le second traitement sont notamment compris dans la liste formée de : une incision, une cautérisation. Avantageusement, le second traitement est différent du premier.

De manière préférentielle, le second traitement est réalisé sans modifier le niveau d'énergie et/ou de forme d'onde délivré par le générateur, par rapport au premier traitement.

À titre d'exemple, le premier traitement peut être une incision en pression des tissus, formé selon le premier mode de mise en oeuvre de l'invention, et représenté sur la figure 3. Dans l'étape suivante, le dispositif est entièrement déplacé en rotation de 90° pour établir les contacts électriques avec les tissus suivant la configuration du troisième mode de mise en oeuvre de l'invention, illustré par la figure 6. Dans l'étape suivante, et sans modifier le niveau d'énergie et/ou de forme d'onde délivré par le générateur, un traitement de cautérisation en pression est réalisé conformément à ce troisième mode de mise en oeuvre. Bien entendu, il est possible d'inverser les deux traitements réalisés, c'est-à-dire réaliser une cautérisation en pression au cours de la première étape ; et suivie d'une incision en pression dans une étape ultérieure.

Dans un second exemple, le premier traitement peut être un traitement d'incision par préhension des tissus selon le deuxième mode de mise en oeuvre de l'invention, et représenté sur la figure 5. Dans l'étape suivante, la seconde électrode est déplacée en rotation de 90° pour établir les contacts électriques avec les tissus suivant la configuration du quatrième mode de mise en oeuvre de l'invention, illustré par la figure 7. Dans l'étape suivante, et sans modifier le niveau d'énergie et/ou de forme d'onde délivré par le générateur, un traitement de cautérisation en préhension est réalisé conformément à ce quatrième mode de mise en oeuvre. Bien entendu, il est possible d'inverser les deux traitements réalisés, c'est-à-dire réaliser une cautérisation en préhension au cours de la première étape ; et suivie d'une incision en préhension dans une étape ultérieure.

Bien entendu, l'invention n'est pas limitée aux modes de mise en oeuvre décrits et on peut y apporter des variantes de réalisation sans sortir du cadre de l'invention tel que défini par les revendications.

Ainsi, bien qu'il soit décrit qu'un des bénéfices de l'invention réside dans la possibilité de réaliser des traitements variés sans nécessairement modifier le niveau d'énergie et/ou la forme d'onde délivrée par le générateur ; l'invention peut tout à fait être mise en oeuvre en modifiant ces paramètres si le chirurgien y trouve un intérêt. A cet effet, le dispositif 1 peut être muni d'une pluralité d'interrupteurs de commande, comme cela a été décrit précédemment.

## Revendications

1. Dispositif électro-chirurgical polyvalent (1) pour le traitement de tissus biologiques comprenant un premier membre (2) comportant à son extrémité avant une première électrode (6) pour former un premier contact électrique avec les tissus et un second membre (3) constitué d'une unique seconde électrode (8) assemblée à un élément de saisie (9), l'unique seconde électrode (8) formant l'extrémité avant du second membre (3), pour former un second contact électrique avec les tissus, le premier et le second membre (2,3) étant reliés par un pivot d'articulation (4) permettant de rapprocher et d'éloigner les membres (2,3) l'un de l'autre ;; la première électrode (6) présentant une section transversale concave, apte à loger en partie la seconde électrode (8) et à mettre en saillie la seconde électrode (8) de la première électrode (6), l'extrémité de la seconde électrode (8) dépassant en longueur l'extrémité de la première électrode (6), lorsque les deux membres (2,3) sont rapprochés l'un de l'autre pour permettre la formation du premier contact électrique sur la surface externe de la première électrode (6) et la formation du second contact électrique sur la partie en saillie de la seconde électrode (8).

2. Dispositif électro-chirurgical polyvalent (1) selon la revendication précédente, dans lequel la seconde électrode (8) n'est pas entièrement logée à l'intérieur de la concavité de la première électrode (6) lorsque les deux membres (2,3) sont rapprochés l'un de l'autre, de sorte que la seconde électrode (8) dépasse en hauteur les bords (11a, 11b) de cette concavité.

3. Dispositif électro-chirurgical polyvalent (1) selon l'une des revendications précédentes dans lequel les axes longitudinaux des électrodes (6, 8) ne sont pas parallèle l'un à l'autre, lorsque les deux membres (2,3) sont rapprochés l'un de l'autre.

4. Dispositif électro-chirurgical polyvalent (1) selon l'une des revendications précédentes dans lequel la section transversale concave est une portion de cercle.

5. Dispositif électro-chirurgical polyvalent (1) selon l'une des revendications précédentes dans lequel la seconde électrode (8) présente une extrémité en forme de lame.

6. Dispositif électro-chirurgical polyvalent (1) selon l'une des revendications 1 à 5 dans lequel la seconde électrode (8) présente une extrémité en forme d'aiguille.

7. Dispositif électro-chirurgical polyvalent (1) selon la revendication 6 dans lequel la seconde électrode (8) en forme de lame est orientée de sorte que sa tranche se loge dans la première électrode (6) à section transversale concave, lorsque les deux membres (2,3) sont rapprochés l'un de l'autre.

8. Dispositif électro-chirurgical polyvalent (1) selon l'une quelconque des revendications précédentes, dans lequel le second membre (3) est pourvu de moyens permettant de déplacer en rotation la seconde électrode (8) autour d'un axe sensiblement défini par l'axe longitudinal du second membre (3).

9. Dispositif électro-chirurgical polyvalent (1) selon la revendication précédente dans lequel les moyens permettant de déplacer en rotation la seconde électrode (8) comprennent une rosace.

10. Dispositif électro-chirurgical polyvalent (1) selon l'une quelconque des revendications précédentes, le dispositif (1) étant muni de moyens de guidage du premier et du second membres (2, 3).

11. Dispositif électro-chirurgical polyvalent (1) selon l'une quelconque des revendications précédentes, dans lequel les électrodes (6,8) sont assemblées à des éléments de saisie (7,9) de manière interchangeable.

## Patentansprüche

1. Vielseitige elektrochirurgische Vorrichtung (1) zur Behandlung von biologischem Gewebe, welche ein erstes Glied (2) enthält, welches an seinem vorderen Ende eine erste Elektrode (6) aufweist, um einen ersten elektrischen Kontakt mit dem Gewebe herzustellen, und ein zweites Glied (3) bestehend aus einer zweiten Einzelelektrode (8), die an ein Greifelement (9) montiert ist, wobei die zweite Einzelelektrode (8) das vordere Ende des zweiten Glieds (3) bildet, um einen zweiten elektrischen Kontakt mit den Geweben herzustellen, wobei das erste und das zweite Glied (2, 3) durch einen Drehzapfen (4) miteinander verbunden sind, der das Annähern und Abspreizen der Glieder (2, 3) voneinander ermöglicht, wobei die erste Elektrode (6) eine konkave Querschnittsfläche aufweist, und in der Lage ist, die zweite Elektrode (8) teilweise aufzunehmen und die zweite Elektrode (8) über die erste Elektrode (6) hinausragen zu lassen, wobei das Ende der zweiten Elektrode (8) in der Länge über das Ende der ersten Elektrode (6) hinausragt, wenn die beiden Glieder (2, 3) einander angenähert werden, um die Herstellung des ersten elektrischen Kontakts an der Außenfläche der ersten Elektrode (6) und die Herstellung des zweiten elektrischen Kontakts an dem vorstehenden Teil der zweiten Elektrode (8) zu ermöglichen.

2. Vielseitige elektrochirurgische Vorrichtung (1) nach dem vorhergehenden Anspruch, in welcher die zweite Elektrode (8) nicht in vollem Umfang in der konkaven Form der ersten Elektrode (6) aufgenommen wird, wenn die beiden Glieder (2, 3) einander angenähert werden, so dass die zweite Elektrode (8) in der Höhe über die Ränder (11a, 11b) dieser konkaven Form hinausragt.

3. Vielseitige elektro-chirurgische Vorrichtung (1) nach einem der vorhergehenden Ansprüche, in welcher die Längsachsen der Elektroden (6, 8) nicht parallel zueinanderstehen, wenn die beiden Glieder (2, 3) einander angenähert werden.

4. Vielseitige elektrochirurgische Vorrichtung (1) nach einem der vorhergehenden Ansprüche, in welcher die konkave Querschnittsfläche ein Kreisabschnitt ist.

5. Vielseitige elektrochirurgische Vorrichtung (1) nach einem der vorhergehenden Ansprüche, in welcher die zweite Elektrode (8) ein klingenförmiges Ende aufweist.

6. Vielseitige elektrochirurgische Vorrichtung (1) nach einem der Ansprüche 1 bis 5, in welcher die zweite Elektrode (8) ein nadelförmiges Ende aufweist.

7. Vielseitige elektrochirurgische Vorrichtung (1) nach Anspruch 6, in welcher die zweite, klingenförmige Elektrode (8) so ausgerichtet ist, dass ihr Rand in der ersten Elektrode (6) mit konkaver Querschnittsfläche aufgenommen wird, wenn die beiden Glieder (2, 3) einander angenähert werden.

8. Vielseitige elektrochirurgische Vorrichtung (1) nach einem der vorhergehenden Ansprüche, in welcher das zweite Glied (3) mit Mitteln versehen ist, die die Rotation der zweiten Elektrode (8) um eine Achse, die im Wesentlichen durch die Längsachse des zweiten Geräts (3) abgegrenzt ist, ermöglicht.

9. Vielseitige elektrochirurgische Vorrichtung (1) nach dem vorhergehenden Anspruch, in welcher die Mittel, die das Rotieren der zweiten Elektrode (8) ermöglichen, eine Rosette enthalten.

10. Vielseitige elektrochirurgische Vorrichtung (1) nach einem der vorhergehenden Ansprüche, wobei die Vorrichtung (1) mit Führungsmitteln für das erste und das zweite Glied (2, 3) versehen ist.

11. Vielseitige elektrochirurgische Vorrichtung (1) nach einem der vorhergehenden Ansprüche, in welcher die Elektroden (6, 8) austauschbar an Greifelemente (7, 9) montiert sind.

## Claims

1. A multi-purpose electrosurgical device (1) for treating biological tissues, comprising a first member (2) comprising at the front end thereof a first electrode (6) to form a first electrical contact with the tissues and a second member (3) consisting of a single second electrode (8) connected to a gripping element (9), the single second electrode (8) forming the front end of the second member (3), to form a second electrical contact with the tissues, the first and second members (2, 3) being connected by an articulation pivot (4) making it possible to bring the members (2, 3) closer to and further away from one another; the first electrode (6) having a concave cross-section, able to partly house the second electrode (8) and to make the second electrode (8) project from the first electrode (6), the end of the second electrode (8) being longer than the end of the first electrode (6), when the two members (2, 3) are brought together to allow the formation of the first electrical contact on the external surface of the first electrode (6) and the formation of the second electrical contact on the projecting part of the second electrode (8).

2. The multi-purpose electrosurgical device (1) according to the preceding claim, wherein the second electrode (8) is not entirely housed in the concavity of the first electrode (6) when the two members (2, 3) are brought together, so that the second electrode (8) is higher than the edges (11a, 11b) of this concavity.

3. The multi-purpose electrosurgical device (1) according to either of the preceding claims, wherein the longitudinal axes of the electrodes (6, 8) are not parallel to one another, when the two members (2, 3) are brought together.

4. The multi-purpose electrosurgical device (1) according to any of the preceding claims, wherein the concave cross-section is a portion of a circle.

5. The multi-purpose electrosurgical device (1) according to any of the preceding claims, wherein the second electrode (8) has an end in the form of a blade.

6. The multi-purpose electrosurgical device (1) according to any claims 1 to 5, wherein the second electrode (8) has an end in the form of a needle.

7. The multi-purpose electrosurgical device (1) according to claim 6, wherein the second electrode (8) in the form of a blade is oriented so that its edge is housed in the first electrode (6) with a concave cross-section, when the two members (2, 3) are brought together.

8. The multi-purpose electrosurgical device (1) according to any of the preceding claims, wherein the second member (3) is provided with means for rotating the second electrode (8) about an axis substantially defined by the longitudinal axis of the second member (3) .

9. The multi-purpose electrosurgical device (1) according to the preceding claim, wherein the means for rotating the second electrode (8) comprise a rose.

10. The multi-purpose electrosurgical device (1) according to any of the preceding claims, the device (1) being provided with means for guiding the first and second members (2, 3).

11. The multi-purpose electrosurgical device (1) according to any of the preceding claims, wherein the electrodes (6, 8) are assembled on gripping elements (7, 9) interchangeably.
